(19) **Europäisches Patentamt / European Patent Office / Office européen des brevets**

(11) **EP 2 163 891 B1**

(12) **FASCICULE DE BREVET EUROPEEN**

(45) Date de publication et mention de la délivrance du brevet:
**22.01.2014 Bulletin 2014/04**

(51) Int Cl.:
*G01N 33/00* (2006.01)     *G01N 27/64* (2006.01)

(21) Numéro de dépôt: **09170325.6**

(22) Date de dépôt: **15.09.2009**

(54) **Dispositif et procédé d'étalonnage d'un capteur à ionisation**

Apparatus and method for the calibration of an ionization sensor

Vorrichtung und Verfahren zur Kalibrierung eines Ionisierungssensors

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(30) Priorité: **15.09.2008 FR 0805042**

(43) Date de publication de la demande:
**17.03.2010 Bulletin 2010/11**

(73) Titulaire: **C & C Ingescience
06370 Mouans Sartoux (FR)**

(72) Inventeur: **Coudert, Jean-François
06370 Mouans Sartoux (FR)**

(74) Mandataire: **Gevers France
41, avenue de Friedland
75008 Paris (FR)**

(56) Documents cités:
**WO-A-91/11014       WO-A-98/01752
WO-A-2007/141862    DE-A1- 19 708 052
JP-A- 2001 155 677   SE-B- 421 460
US-A- 3 742 213      US-A1- 2008 048 107
US-B1- 6 244 093**

## Description

Les composés organiques volatiles (COV) présentent fréquemment des risques importants pour l'homme et l'environnement. Ces risques s'expriment dans une grandeur dite CMR (Cancérigène, Mutagène et Reprotoxique) dont la valeur peut varier de 1 à 3 signifiant respectivement toxique, nocif, irritant et/ou toxique pour l'environnement. Les risques des COV sont en général connus et répertoriés sur leur étiquetage sous forme de symboles oranges, phrases de risque et de sécurité et de pictogrammes décrivant les protections à prendre lors de leur manipulation qui peut nécessiter des masques, des gants, des lunettes, etc.

Il n'est pas rare que ces composés apparaissent dans l'atmopshère avec des concentrations significatives par rapport aux valeurs limites d'exposition (VLE) règlementaires, lorsqu'elles ont été identifiées. La présence des COV est due soit à des manipulations (transvasement, pesée, soutirage, mélange, nettoyage etc.) lors des processus standards, soit à des situations incidentelles ou accidentelles (débordement, renversement, incendie, explosion, etc.)

Malgré cela, ces COV sont couramment utilisés dans l'industrie chimique, la fabrication de pièces en composites, les encres, la pharmacie, la parfumerie, les cosmétiques, les détergents, la mécanique, le nettoyage, etc.

Ces COV sont souvent des solvants, mais ils peuvent aussi être des substances chimiques de synthèse ou naturelles utilisées dans ces industries. On peut citer la réalisation des compositions de peintures, parfums, encres, la fabrication des composites, aussi bien que le nettoyage de pièces mécaniques ou des tissus ou le remplissage du réservoir d'un véhicule à la pompe.

Les risques liés à ces composés, aussi bien pour le public que pour les professionnels, ont alerté les pouvoirs publics qui mettent en place dans le cadre européen une nouvelle réglementation, imposant un enregistrement et des études préalables de toxicité par les entreprises (fabricants ou importateurs) avant toute mise sur le marché lorsque les quantités annuelles dépassent certains seuils. Beaucoup de substances chimiques sont potentiellement concernées.

Il est donc utile de disposer de moyens de mesure ou d'alerte pour détecter et quantifier la présence de COV dans l'atmosphère et pour déterminer l'exposition humaine.

Il existe aujourd'hui principalement deux techniques de mesure.

• La technique par prélèvement et mesure chromatographique en différé

Elle consiste à pomper l'atmosphère contenant le COV à travers une cartouche contenant un matériau poreux capable d'adsorber le COV. Le plus connu de ces matériaux est le charbon actif. Le phénomène physique mis en oeuvre est l'adsorption, c'est-à-dire un phénomène d'adhérence des molécules du COV à la surface des grains du matériau. Cette adsorption demande du temps, de plusieurs dizaines de minutes à plusieurs heures. Ces cartouches sont alors désorbées à chaud dans un solvant liquide connu pour récupérer les molécules initiales. Le liquide obtenu est ensuite analysé avec un chromatographe classique qui permet d'identifier les composants et de restituer les concentrations.

Bien adaptée aux cas ou il y a un mélange inconnu de plusieurs COV, cette technique est néanmoins lourde et nécessite un matériel encombrant et coûteux. Elle présente l'incertitude sur les quantités adsorbées et désorbées et reste inadaptée pour des phénomènes courts Elle est principalement utilisée pour des mesures d'ambiance.

• La technique par mesure directe

Elle est basée sur l'utilisation d'un capteur dans lequel le gaz est aspiré en temps réel. Le capteur contient une lampe à rayons ultra-violet d'une puissance suffisante pour ioniser les atomes des COV. Le flux d'électrons ainsi libéré est capté par des électrodes induisant un courant électrique dont l'intensité dépend directement du nombre d'électrons libérés. La mesure de ce courant est immédiate, le capteur présentant des temps de réponse de l'ordre de la seconde. Après calibration avec un COV de référence, par exemple l'isobutylène, la réponse est fournie directement en PPM (Partie Par Million), voire même en PPB (Partie Par Billion-milliard) relativement à ce COV de référence. L'ensemble du dispositif peut être miniaturisé dans un matériel portable, de la taille d'un terminal téléphonique avec un prix très bas. L'inconvénient, toutefois, est qu'il ne permet pas de distinguer les COV d'un mélange. Il s'applique donc à des mesures à proximité de sources de COV identifiées. Ce capteur s'applique donc à un seul COV ou à un mélange connu de COV. L'avantage de ce capteur à ionisation est de donner une réponse immédiate, très fiable et très précise. Cependant, l'interprétation quantitative en valeur absolue du résultat nécessite de connaître le coefficient de calibration du COV concerné par rapport au COV de référence, sous forme d'un coefficient multiplicateur à appliquer au résultat de la mesure en PPM ou PPB. L'utilisation de ce coefficient ne peut être négligée dans la mesure où il varie de 0,1 à 10.

La méthode connue pour calibrer les capteurs à ionisation consiste à faire réaliser un échantillon composé d'un mélange, dans une proportion connue, d'un gaz neutre, non ionisable par l'appareil, et du COV à calibrer. Comme gaz neutre, on peut considérer l'air pur ou l'azote. L'échantillon, livré dans un emballage métallique, est alors aspiré par le capteur pour déterminer le facteur de calibration. Les fabricants de capteurs fournissent des listes de calibration pré-établies, mais ces listes ne concernent que les substances les plus courantes en chimie industrielle, soit environ un millier sur la dizaine de milliers utilisées par les différentes industries, car le prix de revient de ces échantillons est très élévé. Cette calibration n'est en outre réalisable qu'en temps différé si la substance ne figure pas dans les listes disponibles.

En outre ces échantillons sont réalisés avec des substances de laboratoire d'une très grande pureté (99,999%), alors que les COV utilisés sont de qualité industrielle, avec une pureté inférieure, de 95%, voire moins. Enfin, la calibration des substances naturelles est impossible, car leur composition varie avec leur origine et leur vieillissement. Il est donc fondamental, pour pouvoir tirer pleinement parti des avantages des capteurs à ionisation, de pouvoir effectuer une calibration en temps réel sur le COV réellement concerné.

Les brevets SE421460 et US6244093 décrivent des dispositifs d'étalonnage d'un capteur, comprenant une poche pour accumuler le gaz d'étalonnage.

Les brevets WO91/11014, EP2031383 et US2008/0048107 décrivent des dispositifs pour vaporiser des composés organiques volatiles (COV) dans l'atmosphère par des moyens de chauffage.

La présente invention concerne un dispositif permettant de calibrer le capteur à ionisation en temps réel sur un site de mesure (dans un local ou en atmopshère libre) avec le COV mesuré et de permettre ainsi l'interprétation quantitative immédiate des résultats.

L'objet de l'invention est d'apporter une solution immédiate, simple de mise en oeuvre avec un matériel rustique et peu coûteux pour résoudre d'une manière fiable le problème de calibration.

Mais pour être précise, la calibration doit être réalisée dans la gamme des mesures du réel (en pratique, quelques PPM à quelques centaines de PPM du COV) et l'intégralité du liquide injecté doit se retrouver sous forme gazeuse dans le sac de mesure, ce qui implique que

- un changement de phase incomplet, lié à une vaporisation partielle du type aérosol,
- la présence de recondensation dans l'injecteur ou dans le sac,
- l'inhomogénéité du mélange de gaz dans le sac en terme de concentration ou de température rend le résultat non interprétable.

En d'autres termes, pour étalonner un capteur, on peut lui faire aspirer un mélange échantillon de X molécules de gaz neutre et Y molécules du COV à étalonner sous forme gazeuse, l'étalonnage du mélange consistant à connaître la concentration C en PPM du mélange, soit

$$C = \frac{Y}{X+Y} \simeq \frac{Y}{X} \ ,$$

Y étant très petit devant X, présent dans le sac de mesure.

Après aspiration du mélange, le capteur fournit la valeur Z qui, si le capteur était étalonné, serait égale à C. Le coefficient d'étalonnage K est donc égal à

$$K = \frac{C}{Z}$$

Si on place le capteur en un endroit où on veut précisément connaître la quantité de COV et qu'on lit une valeur T, la quantité réelle R est donc

$$R = K*T$$

Cependant, ce résultat suppose que toute la phase liquide (Y) de l'échantillon d'étalonnage ait été vaporisée et qu'on ait bien

$$Y \ gaz \equiv Y \ liquide \ injecté$$

Pour cela, il faut une vaporisation totale. Or la vaporisation de gouttes n'est pas évidente et la vapeur peut se recondenser.

Ce sont ces difficultés que le demandeur à cherché à éliminer.

A cet effet, l'invention concerne tout d'abord un dispositif d'étalonnage d'un capteur à ionisation destiné à déterminer la présence de composés organiques volatiles (COV) dans l'atmosphère, comprenant une poche étanche de mesure destinée à recevoir un échantillon de référence d'un mélange d'un gaz vecteur et d'un composé volatil, un injecteur pour injecter le gaz vecteur et le composé dans la poche, et des moyens pour introduire le gaz vecteur et le composé dans l'injecteur, caractérisé par le fait qu'il est prévu un four d'injection dans lequel s'étend l'injecteur.

Le gaz vecteur chaud de l'injecteur, du fait du four, favorise la vaporisation du composé volatil.

De préférence, il est prévu une seringue d'injection du composé volatil dans l'injecteur, qui injecte le composé à une vitesse qui permet d'empêcher sa recondensation.

Les avantages de l'invention sont nombreux.

- la réalisation de mesures quasi-instantanées de l'exposition des personnels travaillant dans des industries utilisant des COV, au cours de manipulations courtes mais fortement concentrées afin de réaliser des fiches d'exposition règlementaires rigoureuses;

- la mise en oeuvre de moyens de protection individuelles ou collectives pour ces personnels et le contrôle de l'efficacité dans le temps;
- la quantification d'expositions accidentelles permettant d'informer les services de santé et de mettre en oeuvre immédiatement le traitement approprié;
- les décisions de protection civile suite à des rejets dans l'environnement accidentels ou malveillants;
- le suivi de pollutions potentiellement liées à la mise en oeuvre de traitements agricoles (pesticides, engrais, destructions des nuisibles etc.)

De préférence encore, il est prévu un serpentin autour du four d'injection destiné à être chauffé par le four, à être traversé par le gaz vecteur pour chauffer le gaz et à introduire le gaz vecteur chaud dans l'injecteur.

L'invention concerne également un procédé d'étalonnage d'un capteur à ionisation destiné à déterminer la présence de composés organiques volatiles (COV) dans l'atmosphère, dans lequel on introduit un échantillon de référence d'un mélange d'un gaz vecteur et d'un composé volatile dans une poche étanche de mesure et on fait aspirer le contenu de la poche de mesure par le capteur, caractérisé par le fait que le composé volatile, sous forme liquide, est vaporisé avant d'être introduit dans la poche sous une double action mécanique et thermique.

De préférence, on chauffe le gaz vecteur et on crée un flux de gaz vecteur chaud pour engendrer l'action thermique provoquant la vaporisation. Avantageusement, on soumet le composé volatile liquide à l'action thermique du gaz vecteur chaud en l'y mélangeant à vitesse élevée pour lui appliquer une énergie cinétique engendrant l'effet mécanique qui va assurer une vaporisation complète et empêcher sa recondensation.

L'invention sera mieux comprise à la lecture de la description suivante du dispositif et du procédé d'étalonnage, en référence au dessin en annexe, sur lequel

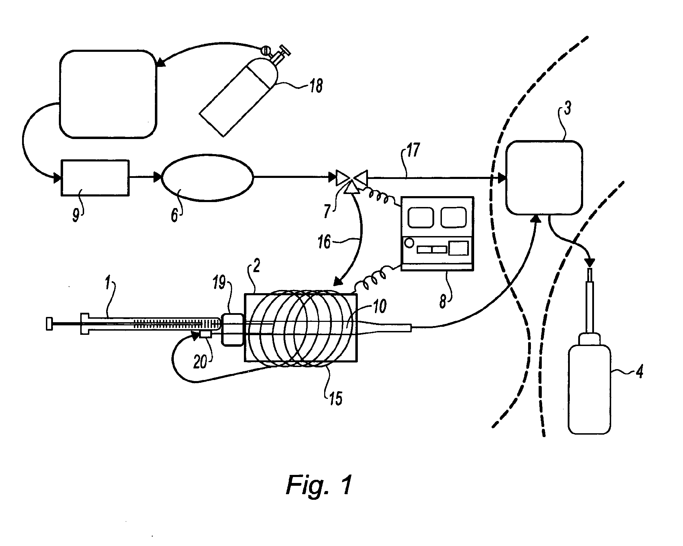

- la figure 1 est un schéma fonctionnel et de principe du dispositif;

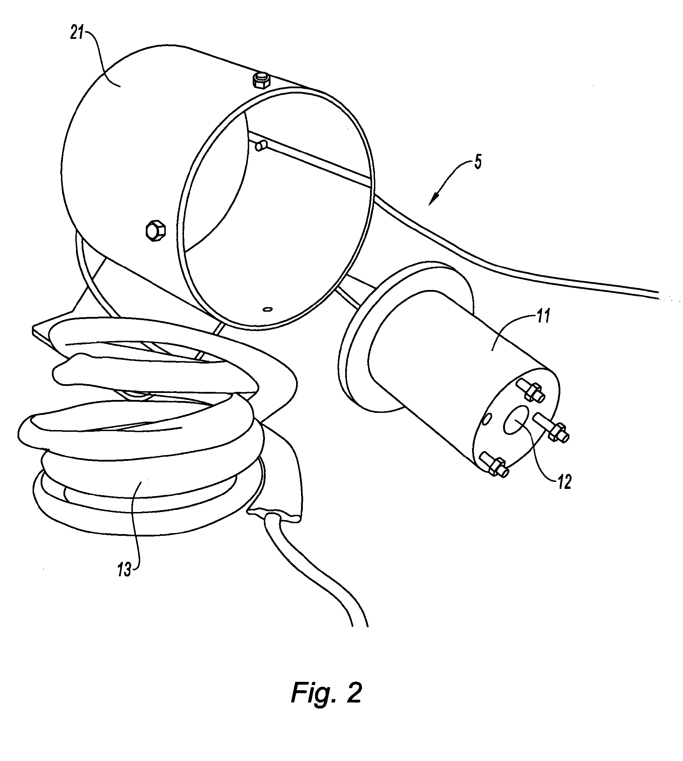

- la figure 2 est une vue en perspective et à l'état démonté du coeur thermique du four d'injection et

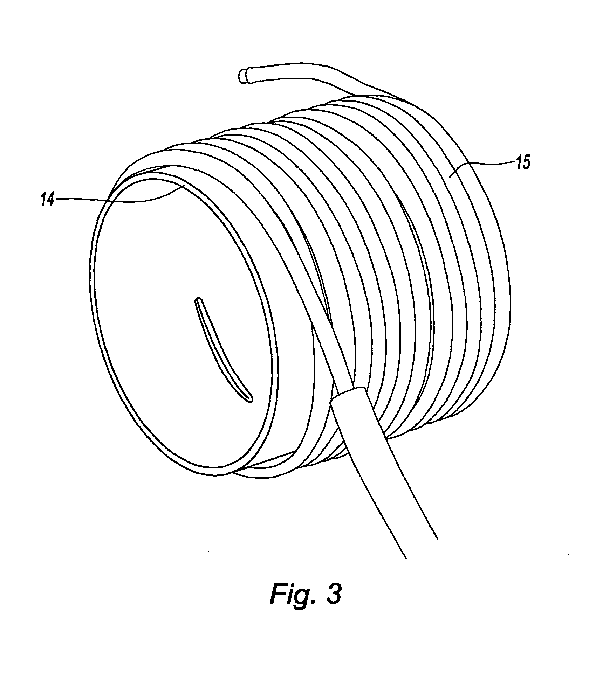

- la figure 3 est une vue en perspective du tube cylindrique recouvert par le serpentin et qui contient le noyau et la résistance.

Le dispositif comporte une seringue d'injection 1 - du type de celles utilisées en chromatographie classique - permettant d'injecter environ un microlitre du COV concerné en phase liquide, un four d'injection 2 permettant de vaporiser ce liquide et de l'injecter avec un volume connu de gaz neutre - dit gaz vecteur - dans un sac de mesure étanche 3 - du type de ceux utilisés pour les prélèvements de gaz - afin de constituer un échantillon de mélange gaz neutre - COV, de concentration connue. L'aspiration de cet échantillon par le capteur 4 permet alors de réaliser la calibration, l'étalonnage.

Le four d'injection 2 comporte a) un coeur thermique 5, b) l'enceinte 14 dans laquelle il est contenu, c) une enceinte 21 de protection de l'ensemble et, pour les éléments périphériques du four, d) un bâti comportant un premier boîtier support de l'enceinte et un débitmètre gaz 6 et qui contient l'alimentation électrique du débitmètre et la sortie vers un ordinateur de contrôle du débitmètre, ainsi qu'une électrovanne 7, un deuxième boîtier 8, qui est un boîtier de contrôle, e) une pompe 9, ici à membrane, pour l'alimentation du gaz vecteur et qui est pourvue en sortie d'une capacité pour atténuer les fluctuations de pression avant le débitmètre 6, f) un ensemble de tuyaux souples de liaison, précisés ci-après, g) un injecteur 10 ici en verre résistant à la chaleur et h) le sac de prélèvement du gaz vecteur et le sac de mesure 3, le tout pouvant être conditionné dans une valise de transport.

Le coeur thermique 5 comporte

- un noyau 11 en cuivre ou laiton massif de forme cylindrique, percé d'un trou 12 selon son axe, capable de recevoir l'injecteur 10,
- une résistance électrique de chauffage 13 bobinée autour du noyau 11 et contrôlée par un thermocouple et un thermostat,
- un tube cylindrique 14 en laiton ou en cuivre contenant l'ensemble, fermé aux deux extrémités par des flasques du même matériau, percé au centre comme le noyau, ces flasques étant solidarisés avec le noyau 11 et le tube 14 par des vis et des soudures permettant

  - le démontage d'un côté, pour la mise en place ou le remplacement éventuel de la résistance 13,
  - par des soudures de l'autre côté, pour assurer une meilleure conductivité entre le noyau et le tube

- un tube capillaire 15 ici en cuivre recuit bobiné en serpentin autour du tube cylindrique 14 et soudé à lui pour préchauffer le gaz vecteur avant l'injection, les extrémités du capillaire dépassant suffisamment du coeur pour être connectée à l'extérieur du four.

Le coeur forme un ensemble compact dont seul sortent les fils d'alimentation de la résistance, les fils du thermocouple, le branchement d'entrée et de sortie du capillaire.

L'enceinte de protection contenant le coeur se présente ici sous forme d'un tube en inox 21 terminé par deux disques cylindriques en matériau isolant du type céramique ou équivalent, solidarisés avec le coeur par des vis. L'espace entre le coeur et l'enceinte est rempli d'un matériau isolant du type fibre de verre.

La protection de l'ensemble est ici constituée d'une tôle en inox cintrée en forme d'arche et percée de trous formant grille pour entourer l'enceinte et protéger l'opérateur tout en contribuant à la dissipation de la chaleur.

Le bâti est ici en tôle d'inox.

L'électrovanne 7 est ici une électrovanne à pincement pour deux tuyaux souples 16, 17 (un fermé, un ouvert) permettant d'éviter la diffusion du COV dans les tuyaux en commutant entre les phases :

- alimentation de l'injection directe du gaz vecteur vers le sac de mesure et isolement du tuyau d'alimentation de l'injecteur par l'aiguille 20,
- alimentation de l'injecteur par l'aiguille 20 et isolement de l'alimentation directe du sac.

L'ensemble de tuyaux souples de liaison, ici en silicone, permet

- de relier l'injecteur 10 à la sortie du débitmètre 6 via l'électrovalve 7,
- l'entrée du débitmètre 6 à la sortie de la pompe 9 d'alimentation en gaz vecteur,
- l'entrée de la pompe d'alimentation 9 à la source de gaz vecteur 18 via un sac de prélèvement étanche ici d'une capacité de 20 litres,
- la vanne du sac de mesure 3 au capteur à ionisation 4.

L'injecteur 10 est ajusté en diamètre pour être positionné dans le coeur thermique en tenant compte des dilatations et dont la longueur permet d'avoir en amont une partie filetée avec un bouchon muni d'un septum 19 à l'extérieur de l'enceinte au travers duquel se font l'injection du gaz vecteur via une aiguille hypodermique 20 et l'injection du COV via la seringue de chromatographie 1. L'autre extrémité dépasse également de l'enceinte avec une partie affinée pour pénétrer dans le sac de mesure via un joint d'étanchéité.

L'aiguille 20, après réchauffement du gaz vecteur dans le serpentin 15, permet de créer un jet (plusieurs mètres par seconde) susceptible de contribuer à la rupture des gouttes par effet mécanique et également d'empêcher la recondensation par balayage des parois de l'injecteur. Il est à noter qu'un effet mécanique trop violent créé par une injection trop rapide avec la seringue 1 provoque la création d'un aérosol qui détruit complètement le procédé.

Le sac de mesure a ici une capacité de 3 litres. Il est pourvu d'une prise type septum où pénètre l'injecteur 10, le septum étant percé au diamètre de la partie effilée de l'injecteur et une prise du type cannelée à laquelle est relié le tuyau d'alimentation du capteur à ionisation 4.

La procédure de mise en oeuvre explique bien le fonctionnement :

1) On commence par positionner l'électrovanne 7 sur le tuyau 17 pour remplir le sac 3 avec environ 40% des Y molécules de gaz vecteur prévues.

2) On oriente l'électrovanne 7 vers le tuyau 16 afin de poursuivre le remplissage du sac 3 via le serpentin 15 et l'aiguille 20.

3) On procède à l'injection des X molécules de COV (en pratique 1 µl) contenu dans la seringue 1 en 20 secondes environ pour éviter la formation d'un jet à la sortie de l'aiguille de la seringue 1.

4) On bascule l'électrovanne 7 vers le tuyau 17 pour terminer le remplissage du sac avec Y molécules du gaz vecteur.

5) Lorsque le sac de mesure contient les X molécules du COV et les Y molécules du gaz vecteur, on isole son alimentation et on aspire la totalité de son contenu avec le capteur de mesure. La lecture de la valeur Z permet de calculer le coefficient d'étalonnage K selon la formule ci-dessus.

L'électrovanne 7 et la seringue d'injection 1 sont asservies à un automate pour permettre une programmation du procédé, tout le dispositif étant piloté par un programme d'ordinateur.

Enfin, on notera que la température du gaz vecteur à la sortie du serpentin 15 peut être fixée indépendamment de la température du four et la vitesse d'injection (débit) dans l'aiguille 20 peut être également ajustée à une valeur spécifique par l'opérateur. L'objectif est de pouvoir moduler l'effet mécanique par rapport à l'effet thermique pour des COV particuliers (forte viscosité, forte tension superficielle, tendance à adhérer sur le verre etc.)

## Revendications

1. Dispositif d'étalonnage d'un capteur à ionisation (4) destiné à déterminer la présence de composés organiques volatiles (COV) dans l'atmosphère, comprenant une poche étanche de mesure (3) destinée à recevoir un échantillon de référence d'un mélange d'un gaz vecteur et d'un composé volatile, un injecteur (10) pour injecter le gaz vecteur et le composé dans la poche (3), et des moyens (1) pour introduire le gaz vecteur et le composé dans l'injecteur (10), le dispositif étant **caractérisé par le fait qu'**il comprend un four d'injection (2) dans lequel s'étend l'injecteur (10).

2. Dispositif d'étalonnage selon la revendication 1, comprenant une seringue (1) d'injection du composé volatile dans l'injecteur (10) à une vitesse qui permet d'empêcher sa condensation.

3. Dispositif d'étalonnage selon l'une des revendications 1 et 2, comprenant un serpentin (15) autour du four (2) destiné à être chauffé par le four, à être traversé par le gaz vecteur pour chauffer le gaz vecteur et à introduire le gaz vecteur chaud dans l'injecteur (10).

**4.** Procédé d'étalonnage d'un capteur à ionisation (4) destiné à déterminer la présence de composés organiques volatiles (COV) dans l'atmosphère, dans lequel on introduit un échantillon de référence d'un mélange d'un gaz vecteur et d'un composé volatile dans une poche étanche de mesure (3) **par un injecteur (10)** et on fait aspirer le contenu de la poche de mesure (3) par le capteur (4), **caractérisé par le fait que** le composé volatile, sous forme liquide, est vaporisé **par un four d'injection (2) dans lequel s'étend l'injecteur (10)** avant d'être introduit dans la poche sous une double action mécanique et thermique.

**5.** Procédé d'étalonnage selon la revendication 4, dans lequel on chauffe le gaz vecteur **au moyen du four d'injection (2)** et on crée un flux de gaz vecteur chaud pour engendrer l'action thermique provoquant la vaporisation.

**6.** Procédé d'étalonnage selon l'une des revendications 4 et 5, dans lequel on soumet le composé volatile liquide à l'action thermique du gaz vecteur chaud en l'y mélangeant à vitesse élevée pour lui appliquer une énergie cinétique engendrant l'effet mécanique qui va permettre une vaporisation complète et empêcher sa recondensation.

**Patentansprüche**

**1.** Vorrichtung zur Kalibrierung eines Ionisationssensors (4), der dazu bestimmt ist, das Vorhandensein flüchtiger organischer Verbindungen (COV) in der Atmosphäre festzustellen, enthaltend eine dichte Messtasche (3), die dazu bestimmt ist, eine Bezugsprobe einer Mischung aus einem Trägergas und einer flüchtigen Verbindung aufzunehmen, einen Injektor (10), um das Trägergas und die Verbindung in die Tasche (3) zu injizieren, und Einrichtungen (1), um das Trägergas und die Verbindung in den Injektor (10) einzuführen, wobei die Vorrichtung durch die Tatsache gekennzeichnet ist, dass sie einen Injektionsofen (2) enthält, in dem sich der Injektor (10) erstreckt.

**2.** Kalibriervorrichtung nach Anspruch 1, enthaltend eine Spritze (1) zur Injektion der flüchtigen Verbindung in den Injektor (10) mit einer Geschwindigkeit, die es ermöglicht, ihre Kondensation zu verhindern.

**3.** Kalibriervorrichtung nach einem der Ansprüche 1 und 2, enthaltend eine Rohrschlange (15) um den Ofen (2) herum, die dazu bestimmt ist, vom Ofen erwärmt zu werden, vom Trägergas durchquert zu werden, um das Trägergas zu erwärmen, und um das warme Trägergas in den Injektor (10) einzuführen.

**4.** Verfahren zur Kalibrierung eines Ionisationssensors (4), der dazu bestimmt ist, das Vorhandensein flüchtiger organischer Verbindungen (COV) in der Atmosphäre festzustellen, wobei eine Bezugsprobe einer Mischung eines Trägergases und einer flüchtigen Verbindung in eine dichte Messtasche (3) durch einen Injektor (10) eingeführt und der Inhalt der Messtasche (3) vom Sensor (4) angesaugt wird, **gekennzeichnet durch** die Tatsache, dass die flüchtige Verbindung in flüssiger Form von einem Injektionsofen (2), in dem sich der Injektor (10) erstreckt, verdampft wird, ehe sie unter einer doppelten mechanischen und thermischen Einwirkung in die Tasche eingeführt wird.

**5.** Kalibrierungsverfahren nach Anspruch 4, bei welchem das Trägergas mittels des Injektionsofens (2) erwärmt und ein warmer Trägergasstrom erzeugt wird, um die die Verdampfung hervorrufende thermische Einwirkung zu erzeugen.

**6.** Kalibrierungsverfahren nach einem der Ansprüche 4 und 5, bei welchem die flüssige flüchtige Verbindung der thermischen Einwirkung des warmen Trägergases ausgesetzt wird, indem sie mit hoher Geschwindigkeit damit vermischt wird, um eine kinetische Energie an sie anzuwenden, die die mechanische Wirkung erzeugt, welche eine vollständige Verdampfung erlaubt und ihre Rekondensation verhindert.

**Claims**

**1.** Device for calibrating an ionisation sensor (4) for determining the presence of volatile organic compounds (VOC) in the atmosphere, comprising a sealed metering pouch (3) for receiving a reference sample of a mixture of a carrier gas and a volatile compound, an injector (10) for injecting the carrier gas and the compound into the pouch (3), and means (1) for introducing the carrier gas and the compound into the injector (10), the device being **characterised in that** it comprises an injection furnace (2) into which the injector (10) extends.

**2.** Calibration device according to claim 1, comprising a syringe (1) for injecting the volatile compound into the injector (10) at a speed which allows said compound to be prevented from condensing.

**3.** Calibration device according to either claim 1 or claim 2, comprising a coil (15) around the furnace (2) which is intended to be heated by the furnace, to be passed through by the carrier gas to heat said carrier gas and to introduce the hot carrier gas into the injector (10).

4. Method for calibrating an ionisation sensor (4) for determining the presence of volatile organic compounds (VOC) in the atmosphere, wherein a reference sample of a mixture of a carrier gas and a volatile compound is introduced into a sealed metering pouch (3) **by an injector (10)** and the contents of the metering pouch (3) are drawn in by the sensor (4), **characterised in that** the volatile compound, in liquid form, is vaporised **by an injection furnace (2) into which the injector (10) extends** before being introduced into the pouch under a double mechanical and thermal action.

5. Calibration method according to claim 4, wherein the carrier gas is heated **by means of the injection furnace (2)** and a flow of hot carrier gas is created to produce the thermal action which causes the vaporisation.

6. Calibration method according to either claim 4 or claim 5, wherein the liquid volatile compound is subjected to the thermal action of the hot carrier gas by mixing it at high speed to apply kinetic energy thereto, causing the mechanical effect which will allow complete vaporisation and prevent said compound from recondensing.

Fig. 1

Fig. 2

14

15

*Fig. 3*

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- SE 421460
- US 6244093 B
- WO 9111014 A
- EP 2031383 A
- US 20080048107 A